(19) Europäisches Patentamt
European Patent Office
Office européen des brevets

(11) Publication number: **0 094 065**
**B1**

(12) # EUROPEAN PATENT SPECIFICATION

(45) Date of publication of patent specification: **20.12.89**

(21) Application number: **83104501.8**

(22) Date of filing: **06.05.83**

(51) Int. Cl.⁴: **C 07 D 215/44**, C 07 D 405/12, A 61 K 31/47, A 61 K 31/55

(54) **2-Aminobenzoic acid derivatives, a process for their preparation and pharmaceutical compositions.**

(30) Priority: **06.05.82 ES 512605**
**18.06.82 ES 514341**

(43) Date of publication of application:
**16.11.83 Bulletin 83/46**

(45) Publication of the grant of the patent:
**20.12.89 Bulletin 89/51**

(84) Designated Contracting States:
**BE CH DE FR GB IT LI NL**

(56) References cited:
**EP-A-0 076 600**
**GB-A-1 234 490**
**GB-A-1 462 676**
**CHIMIE THERAPEUTIQUE, no. 2, March-April 1973, Paris (FR); A.ALLAIS et al.: "Recherche de composés analgésiques non narcotiques. Etude de nouvelles (alcoxycarbonyl-2' phénylamino)-4 quinoléines et de molécules apparentées", pp. 154-168**
**The file contains technical information submitted after the application was filed and not included in this specification**

(73) Proprietor: **FERRER INTERNACIONAL, S.A.**
**Gran Via Carlos III, 94**
**08028 Barcelona (ES)**

(72) Inventor: **Foguet, Rafael**
**Llusanés 8**
**Barcelona (ES)**
Inventor: **Ortiz, José A., Dr.**
**Córcega 429**
**Barcelona (ES)**
Inventor: **Forné, Ernesto, Dr.**
**Felipe de Paz 15**
**Barcelona (ES)**
Inventor: **Sacristán, Aurelio**
**Santa Amelia 2**
**Barcelona (ES)**

(74) Representative: **Kinzebach, Werner, Dr.**
**Patentanwälte Reitstötter, Kinzebach und Partner Sternwartstrasse 4 Postfach 86 06 49**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to 2-aminobenzoic acid derivatives, processes for preparing them and pharmaceutical compositions containing them.

The compounds of the invention are 4-(2-carboxyphenyl)quinolines the carboxy group of which is esterified and which are substituted in the 7-position by halogen atoms or the $CF_3$ group.

Compounds of this kind having pharmacological activity are already known. The GB—A—1 462 676 describes for instance compounds which are substituted at the 7- or 8-position of the quinoline ring system by a trihalomethyl group. The carboxyl group of said compounds is esterified by a group

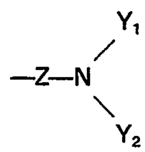

wherein Z is an alkylene group which may be interrupted by an oxygen atom. According to the general disclosure $Y_1$ and $Y_2$ can inter alia represent together an alkylene radical having 4 or 5 carbon atoms. However, in said document there are described expressis verbis only those compounds wherein $Y_1$ and $Y_2$ each represent an alkyl group.

The GB—B—1 234 490 describes similar compounds the carboxyl group of which is esterified by a group $(CH_2)_nR$ (n = 1 or 2) wherein R represents a saturated or unsaturated heterocyclic radical. The examples describe only those compounds wherein R is pyridine or morpholine.

The EP—A—0 076 600 also describes similar compounds the carboxy group of which is esterified by a group $(CH_2)_nR$ with n = 0, 1, 2 and R = 2-oxo-3-oxolanyl or 2-oxo-3-oxozolidinyl. Said document forms part of the state of the art only with respect to Art. 54(3) EPC.

In Chem. Therapeutique No. 2 (1973), 1954—168 there are described quite numerous compounds of the kind described above the carboxyl group of which is esterified by various groups including heterocyclyl groups.

The 2-aminobenzoic acid derivatives of the present invention have the general formula I:

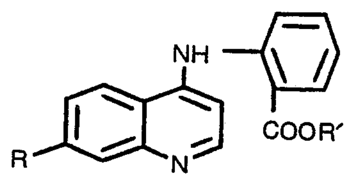

(I)

wherein

R is a halogen atom or a trifluoromethyl group,

R′ represents a radical having the general formula:

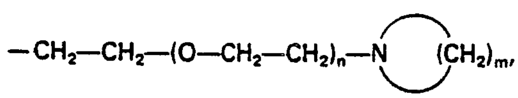

wherein

n is 0 or 1 and

m is 4, 5 or 6;

or a 1(3H)isobenzofuranon-3-yl group.

The meaning halogen atom comprises a fluorine, chlorine, bromine or iodine atom.

The invention also comprises the non-toxic addition salts of these compounds.

The compounds of the present invention are obtained according to the following diagram:

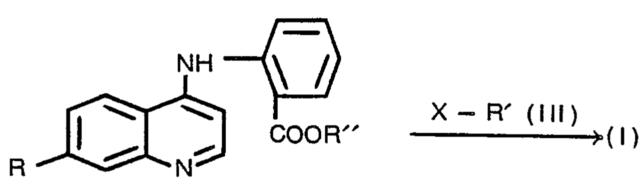


(II)

R and R′ = as defined above

R″ = $C_1$–$C_4$ alkyl, H

X = OH, halogen

In those cases where the compounds of the general formula (II) are esters, i.e. when R″ is a $C_1$—$C_4$ alkyl group, preferably methyl, the reaction is carried out with compounds of the general structure (III) wherein X is OH; then the reaction is properly catalyzed by sodium or sodium hydride and completed at elevated temperatures, preferably under reflux, in an aromatic solvent, such as toluene. The reaction is preferably applied when R′ represents groups of the general structure:

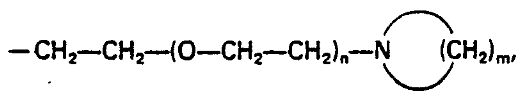

wherein n and m are as defined above. The preferred catalyst is sodium.

On the other hand, in those cases wherein R″ is hydrogen, the reaction is carried out with intermediates of general structure (III) wherein X is a halogen atom, selected from bromine and chlorine. This reaction is preferably applied when R′ is 1(3H)-isobenzofuranon-3-yl. Due to the insolubility of the acids (II, R″ = H) as well as their inorganic salts in most organic solvents, even polar aprotic solvents, the use of salts with organic tertiary amines, such as triethylamine, tri-n-butylamine or the like, is preferred, which achieves solution and consequently facilitates the reaction to a great extent. The reaction then occurs in a high yield using hexamethylphosphoric triamide or N,N-dimethylformamide as solvent at room temperature.

Intermediates of general formula (III), wherein X is OH and R′ is a group of the general structure:

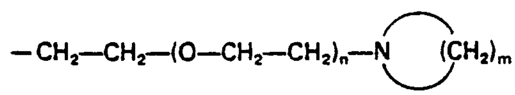

wherein n is 0 and m is as defined above, are obtained according to the methods described by A. Lespagnol and J. Desprey (Bull. Soc. Chim. France, 3, 606—610, 1961). As for respective analogues with n = 1 we have simplified their preparation by modifying the method by H. Najer et al, (Bull. Soc. Chim. France, 3, 355—359, 1958), since it has surprisingly been found that such intermediates are advantageously obtained by reacting the commercially available 2-(2-chloroethoxy)ethanol and the corresponding cyclic amine, thus avoiding laboratory operations under pressure.

Moreover, it was now surprisingly found that the compounds of the general formula (I′) can be advantageously prepared by a new process differing from that described above.

The process for producing 2-amino-benzoic acid derivatives having the general formula (I′):

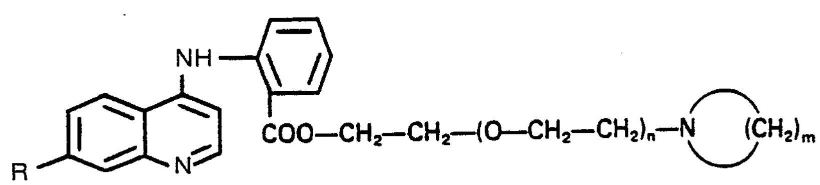

(I′)

wherein R is halogen or trifluoromethyl, n is 0 or 1 and m is 4, 5 or 6 and the additions salts thereof is characterized in that an intermediate of the general formula IV:

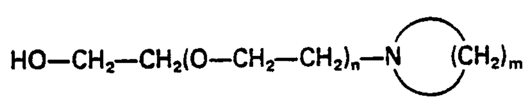

(IV)

wherein n and m are as defined above, is reacted with isatoic anhydride in an aromatic solvent at elevated temperatures to obtain an intermediate of the general formula V:

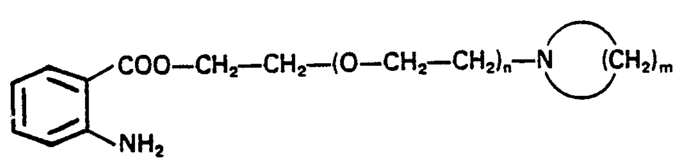

(V)

wherein n and m are as defined above, and the intermediate of the general formula V is then reacted with an intermediate of the general formula VI:

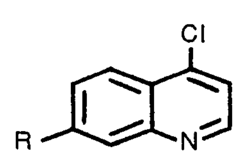

(VI)

wherein R is as defined above, in an aqueous medium, previously acidified with a mineral acid, preferably HCl, at elevated temperatures, preferably under reflux, and the so obtained product is then treated with an alkali metal or alkaline earth metal carbonate or bicarbonate, and optionally the so obtained product is then converted to the corresponding addition salt.

The reaction of isatoic anhydride with an alcohol of formula (IV), obtained as described above, followed by subsequent alkylation with the corresponding 4-chloro-quinolines (VI), leads to the compounds of general formula (I) as described in the following diagram:

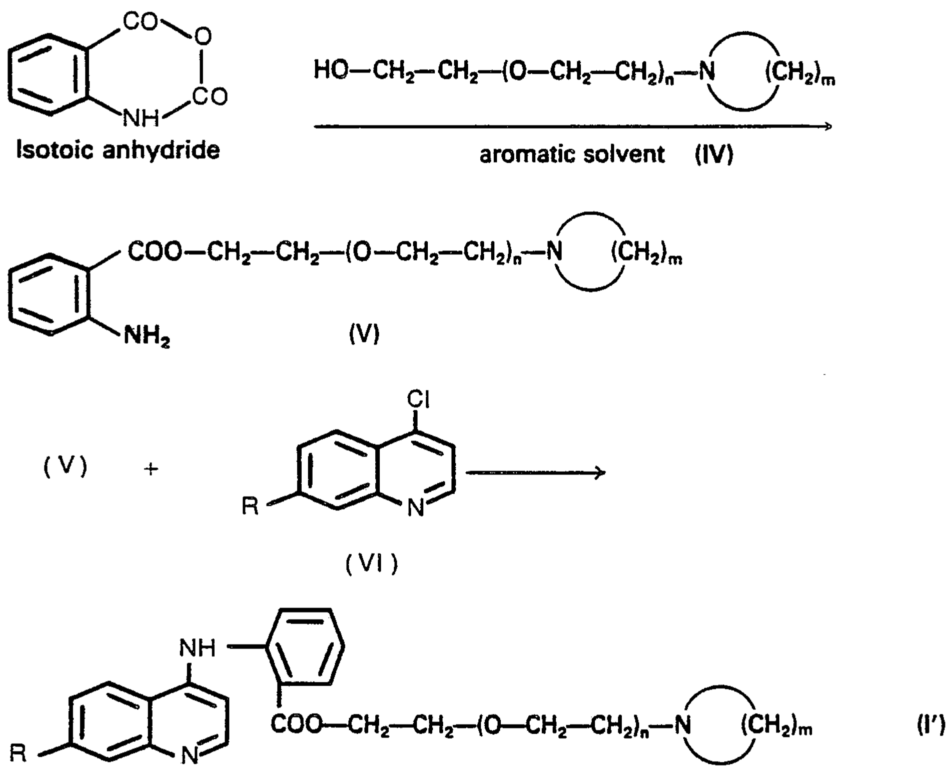


Isotoic anhydride

(IV)

(V)

(V) +

(VI)

(I')

In the above diagram R, n and m for structures (IV), (V) and (VI), are as defined above in (I'). The first reaction is suitably conducted in an aromatic solvent, preferably toluene, at elevated temperatures, preferably at the boiling point of the solvent. The second reaction is suitably conducted in an acidic aqueous solution, at the boiling point of same.

The compounds of the present invention have shown an outstanding analgesic activity, especially by oral route versus the brewer's yeast test in rats as described by Winter et al. (J. Pharm. Exp. Ther., 150, (1), 165—171, 1965). Also these compounds have shown antiinflammatory activity especially by oral route versus the Carrageenin test in rats as described by Winter et al (Proc. Soc. Exp. Biol. Med., 111, 544—547, 1962). The results for the analgesic evaluation are expressed as $ED_{50}$ and those for the antiinflammatory evaluation as the activity rate observed when administering the compounds at a dose of 5 mg/kg. In table 1 the results are compared to those for acetylsalicylic acid, Antrafenine, Floctafenine and Glafenine.

TABLE 1

| Compound (as free base) | R | R′ | Analgesic activity $ED_{50}$ (mg/kg) | Anti-inflammatory activity (%) |
|---|---|---|---|---|
| Example 1 | Cl | 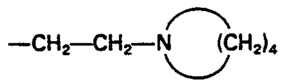 | 38 | 35,6 |
| Example 2 | $CF_3$ | 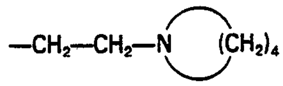 | 2,9 | 38,0 |
| Example 3 | Cl | 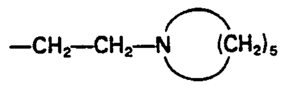 | 67 | 23,1 |
| Example 4 | $CF_3$ | 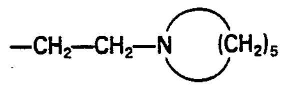 | 3,0 | 23,2 |
| Example 5 | Cl | 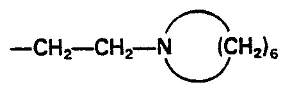 | 5,4 | 17,3 |
| Example 6 | $CF_3$ | 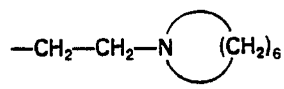 | 7,0 | 27,9 |
| Example 7 | Cl | 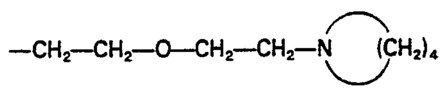 | 18,5 | 5,1 |
| Example 8 | $CF_3$ | 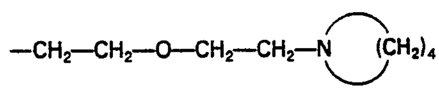 | 14,7 | 26,2 |
| Example 9 | Cl | 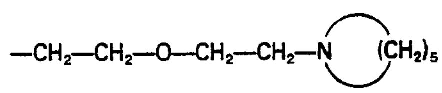 | 21,7 | 33,2 |
| Example 11 | Cl | 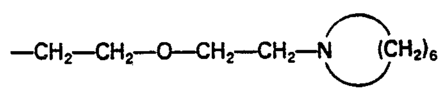 | 13,3 | 21,2 |
| Example 12 | $CF_3$ | 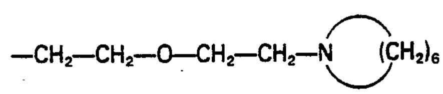 | 6,6 | 28,8 |
| Example 14 | Cl | 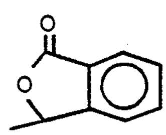 | 71 | 26,2 |
| Example 15 | $CF_3$ | 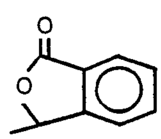 | 26 | 38,4 |
| Acetylsolicylic acid | | | 73 | 23,2 |
| Antrafenine(2-[4-(trifluoro-m-tolyl)-1-piperazinyl]-ethyl-N-(7-trifluoromethyl-4-quinolyl)anthranilate | | | 43 | 3,6 |
| Floctafenine(N-[8-(trifluoromethyl)-4-quinolyl)-anthranylic acid 2,3-dihydroxypropyl ester) | | | 2,2 | 21,6 |
| Glaphenine(N-(7-chloro-4-chinolyl)-anthranylic acid 2,3-dihydroxypropyl ester) | | | 26,5 | 25,6 |

Moreover, the above compounds have shown a low toxicity which makes them useful in therapy. In Table 2 $LD_{50}$-values for some of the most active compounds are compared to those for Glafenine. Evaluation was made according to the method by Reed-Münch as modified by Pizzi (Human & Biology, 22(3), 151—190, 1950) by oral administration to mice.

TABLE 2

| Compound (as free base) | R | R' | $LD_{50}$ (mg/kg) |
|---|---|---|---|
| Example 1 | Cl | 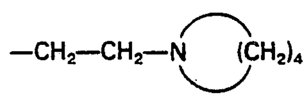 | 2000 |
| Example 2 | $CF_3$ | 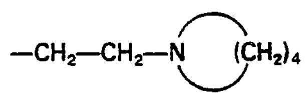 | 630 |
| Example 3 | Cl | 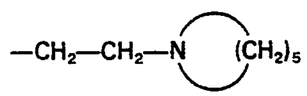 | >2000 |
| Example 4 | $CF_3$ | 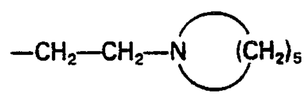 | 635 |
| Glaphenine | | | 2100 |

The compounds of the present invention mixed with pharmaceutically acceptable carriers can be administered by the oral route in form of tablets, capsules, syrups, solutions, etc., by injectable route and by rectal route, at daily doses ranging from 200 to 1000 mg.

A number of examples will now be described in non-limitative manner to illustrate the invention and naturally larger quantities than those indicated can be used in industry.

Example 1

2-Pyrrolidinylethyl 2-(7-chloro-4-quinolyl)aminobenzoate

(I, R = Cl, R' = 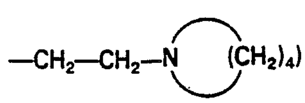)

A mixture containing 18.76 g of methyl 2-(7-chloro-4-quinolyl)-aminobenzoate (II, R = Cl, R'' = $CH_3$) (A. Allain et al: "Chim. Ther.", *2*, 65—70, 1966) and 10.29 g of 1-(2-hydroxyethyl)pyrrolidine in 150 ml of toluene is heated in an oil-bath until about 30 ml of solvent are distilled off; it is allowed to cool to 50°C and a small scrap of clean sodium is added. Temperature is gradually increased in order to distill the formed methanol while replacing the toluene which is also removed. Eight hours later and after having added, at identical intervals, two further scraps of sodium, the mixture is subjected to reflux for 2 hours; it is observed by thin-layer chromatography (silicagel; eluent, chloroform-methanol 9:1) that the transesterification is just complete. The toluene is evaporated under vacuum, the residue is dissolved in 500 ml of chloroform and then filtered off. The organic extract is washed with water, 10% sodium hydroxide solution, and water again. After the solvent is evaporated, the solid residue weighs 22.1 g (yield: 94%). By recrystallization from di-isopropyl ether, 14.5 g (yield: 62%) of a pale-yellow solid are obtained; m.p. 102—104°C, correct elemental analysis.

IR Spectrum (KBr), $cm^{-1}$: 3280, 300—2740, 1670, 1600, 1570, 1525, 1450, 1370, 1245, 1160, 1080, 875, 860, 745.

Example 2

2-Pyrrolidinylethyl 2-(7-trifluoromethyl-4-quinolyl)-amino-benzoate

(I, R = $CF_3$, R' = 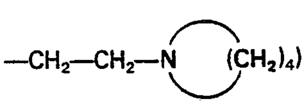)

By treating 19.5 g of methyl 2-(7-trifluoromethyl-4-quinolyl)aminobenzoate (II, R = $CF_3$, R'' = $CH_3$) and 9.59 g 1-(2-hydroxyethyl)pyrrolidine in 145 ml of toluene and in the presence of sodium as described in

example 1, 19.2 g of a crude material are isolated. Purification through silicagel, elution with a mixture of highly-polar chloroform-methanol, and treatment of the product thus purified with hexane, yields 11.6 g (56%) of white-yellowish solid; m.p. 63—65°C, correct elemental analysis C, H, N and F.

IR Spectrum (KBr), $cm^{-1}$: 3400, 3240, 3000—2740, 1670, 1595, 1580, 1530, 1455, 1320, 1250, 1155, 1120, 905, 750.

Example 3

2-Piperidinylethyl-2-(7-chloro-4-quinolyl)-aminobenzoate

(I, R = Cl, R' = $—CH_2—CH_2—N$ $(CH_2)_5$)

By treating 15.54 g of methyl 2-(7-chloro-4-quinolyl)aminobenzoate (II, R = Cl, R'' = $CH_3$), and 10.0 g of 1-(2-hydroxyethyl)piperidine in 100 ml of toluene and in the presence of sodium as described in Example 1, 17.0 g of a crude material are isolated. By recrystallization from diisopropyl ether with active carbon, 12.8 g (yield: 63%) of pale-yellow solid are obtained; m.p. 100—101°C, correct elemental analysis.

IR Spectrum (KBr), $cm^{-1}$: 3300, 3030, 2920, 2900—2760, 1680, 1600, 1575, 1520, 1450, 1250, 1160, 1065, 865, 740.

Example 4

2-Piperidinylethyl 2-(7-trifluoromethyl-4-quinolyl)aminobenzoate

(I, R = $CF_3$, R' = $—CH_2—CH_2—N$ $(CH_2)_5$

By treating 17.31 g of methyl 2-(7-trifluoromethyl-4-quinolyl)-aminobenzoate (II, R = $CF_3$, R'' = $CH_3$) and 9.56 g 1-(2-hydroxyethyl)-piperidine in 130 ml of toluene and in the presence of sodium as described in example 1, 17.9 g of a crude material are isolated. Purification through silicagel and recrystallization from diisopropyl ether enables to isolate 13.3 g (yield: 60%) of white solid; m.p. 78—80°C, correct C, H, N, F elemental analysis.

IR Spectrum (KBr), $cm^{-1}$: 3440, 3320, 3000—2700, 1695, 1605, 1580, 1530, 1380, 1325, 1255, 1150, 1130, 825, 740.

Example 5

2-Homopiperidinylethyl 2-(7-chloro-4-quinolyl)aminobenzoate

(I, R = Cl, R' = $—CH_2—CH_2—N$ $(CH_2)_6$)

By treating 18.76 g of methyl 2-(7-chloro-4-quinolyl)aminobenzoate (II, R = Cl, R'' = $CH_3$) and 12.72 g of 1-(2-hydroxyethyl)homopiperidine in 150 ml of toluene and in the presence of sodium as described in Example 1, 22.07 g of a crude material are isolated. By recrystallization from diisopropyl ether, 15.5 g (yield: 61%) of a yellowish solid are obtained; m.p. 76.5—78°C, correct C, H, N, Cl elemental analysis.

IR Spectrum (KBr), $cm^{-1}$: 3280, 300—2760, 1675, 1605, 1570, 1520, 1450, 1320, 1240, 1160, 1080, 875, 745.

Example 6

2-Homopiperidinylethyl 2-(7-fluoromethyl-4-quinolyl)aminobenzoate

(I, R = $CF_3$, R' = $—CH_2—CH_2—N$ $(CH_2)_6$)

By treating 17.31 g of methyl 2-(7-trifluoromethyl-4-quinolyl)-aminobenzoate (II, R = $CF_3$, R'' = $CH_3$), 10.5 g of 1-(2-hydroxyethyl)homopiperidine in 130 ml of toluene and in the presence of sodium as described in Example 1, 20.6 g of a crude material are isolated. The recrystallization from diisopropyl ether enables to isolate 13.7 g (yield: 60%) of a whitish solid; m.p. 72—75°C, correct C, H, N, F elemental analysis.

IR Spectrum (KBr), $cm^{-1}$: 3420, 3000—2790, 1680, 1605, 1580, 1530, 1450, 1380, 1320, 1250, 1155, 1025, 900, 745.

Example 7

2-(2-Pyrrolidinoethoxy)ethyl 2-(7-chloro-4-quinolyl)aminobenzoate

(I, R = Cl, R' = $—CH_2—CH_2O—CH_2—CH_2—N$ $(CH_2)_4$)

By treating 18.76 g of methyl 2-(7-chloro-4-quinolyl)aminobenzoate (II, R = Cl, R'' = $CH_3$) and 14.14 g of 2-(2-pyrrolidinoethoxy)ethanol in 150 ml of toluene and in the presence of sodium as described in Example 1, 16.2 g of a crude material are isolated. Purification through silicagel and crystallization from hexane enables to isolate 10.81 g (yield: 41%) of a yellow solid; m.p. 60—63°C, correct C, H, N, Cl elemental analysis.

IR Spectrum (KBr), $cm^{-1}$: 3290, 3000—2890, 1670, 1610, 1575, 1525, 1455, 1250, 1085, 860, 745.

Example 8

2-(2-Pyrrolidinoethoxy)ethyl 2-(7-trifluoromethyl-4-quinolyl)aminobenzoate

(I, R = $CF_3$; R' = 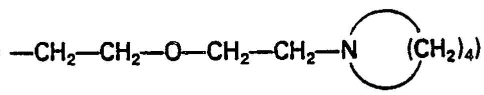

By treating 17.31 g of methyl 2-(7-trifluoromethyl-4-quinolyl)aminobenzoate (II, R = $CF_3$, R'' = $CH_3$), and 11.78 g of 2-(2-pyrrolidinoethoxy)ethanol in 150 ml of toluene and in the presence of sodium as described in Example 1, 13.9 g of a crude material are isolated under nitrogen atmosphere. Purification through silicagel and crystallization from hexane enables to isolate 6.95 g (yield: 30%) of a white solid; m.p. 58—61°C, correct C, H, N, F elemental analysis.

IR Spectrum (KBr), $cm^{-1}$: 3400, 3240, 3000—2780, 1675, 1595, 1585, 1530, 1455, 1380, 1325, 1250, 1160, 1115, 750.

Example 9

2-(2-Piperidinoethoxy)ethyl 2-(7-chloro-4-quinolyl)aminobenzoate

(I, R = Cl, R' = 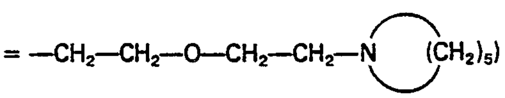

By treating 18.76 g of methyl 2-(7-chloro-4-quinolyl)aminobenzoate (II, R = Cl, R'' = $CH_3$), and 15.38 g of 2-(2-piperidinoethoxy)ethanol in 150 ml of toluene and in the presence of sodium as described in Example 1, 20.32 g of a resinous crude material are isolated. Subsequent treatment with 50 ml of hexane and decantation results in a solid material which is recrystallized from diisopropyl ether. 12.2 g (yield: 45%) of a yellowish solid are obtained; correct C, H, N, Cl elemental analysis.

IR Spectrum (KBr), $cm^{-1}$: 3440, 3240, 3000—2660, 1675, 1600, 1580, 1530, 1450, 1375, 1255, 1130, 1085, 950, 745.

Example 10

2-(2-Piperidinoethoxy)ethyl 2-(7-trifluoromethyl-4-quinolyl)aminobenzoate

(I, R = $CF_3$, R' = 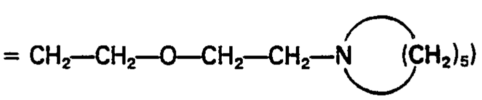

By treating 17.31 g of methyl 2-(7-trifluoromethyl-4-quinolyl)aminobenzoate (II, R = $CF_3$, R'' = $CH_3$), and 15.4 g of 2-(2-piperidinoethoxy)ethanol in 150 ml of toluene and in the presence of sodium as described in Example 1, 22.1 g of a crude material are isolated. Upon subsequent treatment with hexane (50 ml) and recrystallization of the insolute from diisopropyl ether, 10.4 g (yield: 43%) of a yellowish solid are obtained; m.p. 81—83°C, correct C, H, N, F elemental analysis.

IR Spectrum (KBr), $cm^{-1}$: 3290, 3000—2700, 1675, 1610, 1580, 1530, 1455, 1250, 1150, 745.

Example 11

2-(2-Homopiperidinoethoxy)ethyl 2-(7-chloro-4-quinolyl)aminobenzoate

(I, R = Cl, R' = 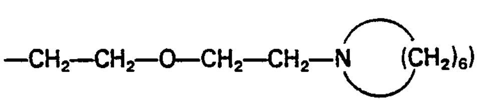

By treating 18.76 g of methyl 2-(7-chloro-4-quinolyl)aminobenzoate (II, R = Cl, R'' = $CH_3$) and 16.63 g of 2-(2-homopiperidinoethoxy)ethanol in 150 ml toluene and in the presence of sodium, as described in Example 1, 29.3 g of a resinous crude material are isolated. Subsequent treatment in 50 ml of hexane and decantation results in a solid material which is consecutively recrystallized from hexane and diisopropylether. 13.3 g (yield: 49%) of a yellowish solid are obtained; m.p. 62—64°C, correct C, H, N, Cl elemental analysis.

IR Spectrum (KBr), $cm^{-1}$: 3240, 3000—2700, 1675, 1600, 1570, 1520, 1445, 1245, 1080, 875, 740.

Example 12

2-(2-Homopiperidinoethoxy)ethyl 2-(7-fluoromethyl-4-quinolyl)aminobenzoate

(I, R = $CF_3$, R' = 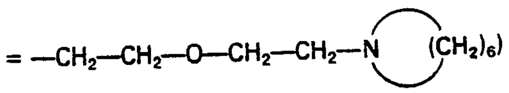)

By treating 17.31 g of methyl 2-(7-trifluoromethyl-4-quinolyl)aminobenzoate (II, R = $CF_3$, R'' = $CH_3$), 13.85 g of 2-(2-homopiperidino-ethoxy)ethanol, 150 ml of toluene and in the presence of sodium, as described in Example 1, 20.3 g of a resinous crude material are isolated. Subsequent crystallization in n-pentane and diisopropyl ether yields 12.1 g (47.5%) of a white-yellowish solid; m.p. 53—56°C, correct C, H, N, F elemental analysis.

IR Spectrum (KBr), $cm^{-1}$: 3260, 3000—2700, 1670, 1595, 1580, 1520, 1445, 1375, 1240, 1155, 1105, 740.

Example 13 (Intermediate)

a) 2-(2-Homopiperidinoethoxy)ethanol

To a mixture, containing 59.81 g of hexamethylenimine, and 50.66 g of sodium bicarbonate in 1 liter of absolute ethanol is added dropwise 56.06 g of 2-(2-chloroethoxy)ethanol with stirring, and the reaction mixture is then subjected to reflux for 24 hours. It is cooled, the insoluble is filtered off, the ethanol is distilled under vacuum and the residue (82.1 g) is dissolved in ethyl ether, filtered again and, under cooling, 55 ml of a 10N ethanolic hydrochloric acid (g) solution are added. After 30 minutes the precipitated hydrochloride is filtered and dried, thus obtaining 80 g (yield: 70%) of a white solid; m.p. 96—99°C, correct analysis.

The hydrochloride thus obtained in 225 ml of water is brought to a highly-basic pH by addition of concentrated $NH_4OH$; it is extracted with methylene chloride, dried and the solvent is evaporated. By distillation under vacuum of the residue, 68.5 g (yield: 61%) of a colorless liquid are obtained; b.p. 125°C/2.5 torr, $n_D^{20}$ = 1.4819.

IR Spectrum (film), $cm^{-1}$: 3400, 3000—2600, 1450, 1350, 1320, 1130, 1060, 890.

By operating as described in a) for 2-(2-pyrrolidinoethoxy)ethanol and 2-(2-piperidinoethoxy)ethanol, both compounds are obtained with the following characteristics:

b) 2-(2-pyrrolidinoethoxy)ethanol: $n_D^{20}$ = 1.4695; m.p. (HCl), 104—108°C, IR-Spectrum (film), $cm^{-1}$: 3400, 3000—2700, 1460, 1350, 1125, 1060, 880.

c) 2-(2-piperidinoethoxy)ethanol: $n_D^{20}$ = 1.4765; m.p. (HCl), 114—118°C, IR-Spectrum (film), $cm^{-1}$: 3500, 3000—2600, 1440, 1340, 1300, 1260, 1130, 1050, 1030, 850, 750.

Example 14

1(3H)-isobenzofuranon-3-yl 2-(7-chloro-4-quinolyl)aminobenzoate

(I, R=Cl, R' = 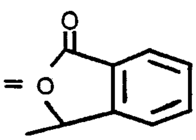 )

To a suspension of 9.82 g of 2-(7-chloro-4-quinolyl)aminobenzoic acid (II, R = Cl, R'' = H) in 200 ml of hexamethylphosphorotriamide, 4.16 g of triethylamine are added and the mixture is stirred until the whole acid is dissolved. Then 8.75 g of 3-bromo-1(3H)-isobenzofurane are added with stirring for 48 hours at room temperature. The mixture is poured onto 1.5 l of water, then brought to stand for a few hours, filtered and repeatedly washed with water. After drying 15 g are obtained which are recrystallized from acetonitrile yielding 11.9 g (84%) of a yellow solid, m.p. 213—215°C, correct C, H, N, Cl elemental analysis.

IR Spectrum (KBr), $cm^{-1}$: 3300, 3040, 1780, 1695, 1610, 1585, 1525, 1455, 1240, 1070, 970, 745.

Example 15

1(3H)-isobenzofuranon-3-yl 2-(7-trifluoromethyl-4-quinolyl)aminobenzoate

(I, R=$CF_3$, R'= 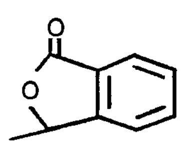 )

By treating 9.55 g of 2-(7-trifluoromethyl-4-quinolyl)aminobenzoic acid (II, $CF_3$, R'' = H) in 200 ml of hexamethylphosphorotriamide, 3.65 g of triethylamine and 7.69 g of 3-bromo-1(3H)-isobenzofuranon as described in Example 14, 11.3 g (yield: 85%) of a yellow solid are isolated after recrystallization from acetonitrile, m.p. 218—220°C, correct C, H, N, F elemental analysis.

IR Spectrum (KBr), $cm^{-1}$: 3300, 3010, 1780, 1700, 1580, 1540, 1460, 1360, 1245, 1155, 1125, 980, 750.

Example 16

2-Pyrrolidinylethyl 2-(7-chloro-4-quinolyl)aminobenzoate (I', R = Cl, n = 0, m = 4)

A) 2-Pyrrolidinylethyl anthranilate (V, n = 0, m = 4):

A mixture with 15,07 g of isatoic anhydride and 7.96 g of 1-(2-hydroxyethyl)pyrrolidine in 150 ml of toluene is subjected to reflux with stirring for 3 hours. Active carbon is added and then filtered off under heating. The toluene is evaporated under reduced pressure, the residue is taken up in ethyl ether, the insoluble is filtered off and the organic solution, under cooling, is extracted twice with 75 ml of a 6N hydrochloric acid solution. The acid extracts are brought, under cooling, to a basic pH with concentrated ammonium hydroxide solution and the oil thus separated is extracted with ethyl ether, washed and dried. After the solvent is evaporated, the residue, a yellow oil with $n_D^{20}$ = 1.572 and correct elemental analysis weighs 13.8 g (yield: 85%). It is distillable in ball furnace; b.p. 185—190°/$10^{-3}$—$10^{-4}$ torr.

IR Spectrum (film), $cm^{-1}$: 3470, 3360, 3260, 2880, 2800, 1680, 1610, 1580, 1480, 1450, 1285, 1235, 1150, 1100, 740.

B) Reaction with 4,7-dichloroquinoline (VI, R = Cl):

11.71 g of 2-pyrrolidinoethyl anthranilate, obtained as described above, and 9.90 g of 4,7-dichloroquinoline in 50 ml of 2N hydrochloric acid are subjected to reflux with stirring for 3 hours. The mixture is allowed to cool, the insoluble is filtered off, and the filtrate is brought to a strongly-basic pH with saturated sodium carbonate solution. The separated solid is filtered off, washed repeatedly and dried. 11.5 g (yield: 58%) of 2-pyrrolidinoethyl 2-(7-chloro-4-quinolyl) aminobenzoate (I', R = Cl, n = 0, m = 4) are obtained by recrystallization, m.p. 102—104°C, correct elemental analysis.

IR Spectrum (KBr), $cm^{-1}$: 3280, 3000—2740, 1670, 1600, 1570, 1525, 1450, 1370, 1245, 1160, 1080, 875, 860, 745.

Example 17

2-Pyrrolidinylethyl 2-(7-trifluoromethyl-4-quinolyl)aminobenzoate (I', R = $CF_3$, n = 0, m = 4)

A mixture with 96.2 g of 2-pyrrolidinylethyl anthranilate and 95.1 g of 4-chloro-7-trifluoromethylquinoline in 410 ml of 2N hydrochloric acid is subjected to reflux with stirring for 3 hours. 127 g of a whitish solid are obtained according to example 16. Recrystallization from n-pentane yields 95.3 g (54%) of a white-yellowish solid, m.p. 65—68°C, correct elemental analysis, which is 2-pyrrolidinylethyl 2-(7-trifluoromethyl-4-quinolyl)aminobenzoate (I', R = $CF_3$, n = 0, m = 4).

IR Spectrum (KBr), $cm^{-1}$: 3400, 3240, 3000—2740, 1670, 1595, 1580, 1530, 1455, 1320, 1155, 1120, 905, 750.

Example 18

2-Piperidinylethyl 2-(7-chloro-4-quinolyl)aminobenzoate (I', R = Cl, n = 0, m = 5)

A) 2-Piperidinylethyl anthranilate (V, n = 0, m = 5):

22.95 g of isatoic anhydride are reacted with 13.59 g of 1-(2-hydroxyethy)piperidine in 220 ml of toluene. 20.53 g (yield: 75%) of a yellow oil are isolated according to example 16; $n_D^{20}$ = 1.567, correct elemental analysis. It may be purified by careful distillation in ball furnace at 175—190°C/$10^{-3}$—$10^{-4}$ torr.

IR Spectrum (KBr), $cm^{-1}$: 3480, 3370, 2940, 2790, 1690, 1620, 1590, 1455, 1290, 1245, 1160, 1105, 750.

B) Reaction with 4,7-dichloroquinoline (VI, R = Cl):

12.41 g of 2-piperidinylethyl anthranilate (as described above) and 9.90 g of 4,7-dichloroquinoline in 50 ml 2N hydrochloric acid are subjected to reflux for 3 hours. As described in Example 16, and by recrystallization from diisopropyl ether, 11.4 g (yield: 56%) of 2-piperidinylethyl 2-(7-chloro-4-quinolyl)aminobenzoate are obtained (I', R = Cl, n = 0, m = 5); m.p. 101—102°C, correct elemental analysis.

Example 19

2-Piperidinylethyl 2-(7-trifluoromethyl-4-quinolyl)aminobenzoate (I', R = $CF_3$, n = 0, m = 5)

By treating 2-piperidinylethyl anthranilate (V, n = 0, m = 5) (51 g) and 4-chloro-7-trifluoromethylquinoline (47.6 g) as described in Example 16, and by recrystallization from n-pentane, 51.9 g (yield: 57%) of 2-piperidinylethyl 2-(7-trifluoromethyl-4-quinolyl)aminobenzoate (I', R = $CF_3$, n = 0, m = 5) are obtained; correct elemental analysis.

IR Spectrum (KBr), $cm^{-1}$: 3440, 3320, 3000—2760, 1695, 1605, 1580, 1530, 1380, 1325, 1255, 1150, 1130, 825, 740.

Example 20

2-Homopiperidinylethyl 2-(7-chloro-4-quinolyl)aminobenzoate (I', R = Cl, n = 0, m = 6)

A) 2-Homopiperidinylethyl anthranilate (V, n = 0, m = 6):

7.54 g of isatoic anhydride are reacted with 5.36 g of 1-(2-hydroxyethyl)-homopiperidine in 100 ml of toluene. 9.46 g of a viscous yellow oil are isolated according to Example 16. By distillation in ball furnace 6.8 g (yield: 70%) of oil are obtained at 180—195°C/$10^{-3}$—$10^{-4}$ torr, $n_D^{20}$ = 1.570, correct C, H, N elemental analysis.

IR Spectrum (film), $cm^{-1}$: 3470, 3360, 1680, 1610, 1580, 1480, 1450, 1290, 1240, 1155, 1105, 750, 700.

B) Reaction with 4,7-dichloroquinoline (VI, R = Cl):

By treating 2-homopiperidinylethyl anthranilate (5.4 g) as above described and 4,7-dichloroquinoline (4.1 g) according to example 16, and after recrystallization, 5.1 g (yield: 55%) of 2-homopiperidinylethyl 2-(7-chloro-4-quinolyl)aminobenzoate (I', R = Cl, n = 0, m = 6) are obtained; m.p. 76.5—78°C, correct elemental analysis.

IR Spectrum (KBr), $cm^{-1}$: 3280, 3000—2760, 1675, 1605, 1570, 1520, 1450, 1320, 1240, 1160, 1080, 875, 745.

Example 21

2-Homopiperidinylethyl 2-(7-trifluoromethyl-4-quinolyl)aminobenzoate (I', R = $CF_3$, n = 0, m = 6)

By treating 2-homopiperidinylethyl anthranilate (5.40 g) as described in Example 26, and 4-chloro-7-trifluoromethylquinoline (4.75 g) as described in Example 16, and after recrystallization from diisopropyl ether, 5.4 g (yield: 58%) of 2-homopiperidinylethyl 2-(7-trifluoromethyl-4-quinolyl)aminobenzoate (I', R = $CF_3$, n = 0, m = 6) are obtained; m.p. 72—75°C correct elemental analysis.

IR Spectrum (KBr), $cm^{-1}$: 3420, 3240, 3000—2790, 1680, 1605, 1580, 1530, 1450, 1380, 1320, 1250, 1155, 1025, 900, 745.

Example 22

2-(2-Pyrrolidinoethoxy)ethyl 2-(7-chloro-4-quinolyl)aminobenzoate (I', R = Cl, n = 1, m = 4)

A) 2-(2-Pyrrolidinoethoxy)ethyl anthranilate (V, n = 1, m = 4):

11.0 g of 2-(2-pyrrolidinoethoxy)ethanol are reacted with 15.07 g of isatoic anhydride in 170 ml of toluene. 14.5 g (yield: 76%) of a viscous yellow oil are isolated according to Example 16. Then, it is purified by careful distillation in ball furnace at 175—190°C/$10^{-4}$—$10^{-3}$ torr, as a heavy oil; $n_D^{20}$ = 1.5589, correct C, H, N elemental analysis.

IR Spectrum (film), $cm^{-1}$: 3460, 3380, 3010—2700, 1685, 1615, 1585, 1485, 1455, 1290, 1245, 1160, 1100, 860, 750.

B) Reaction of 4,7-dichloroquinoline (VI, R = Cl)

By treating 2-(2-pyrrolidinoethoxy)ethyl anthranilate (7.72 g) as described above, and 4,7-dichloroquinoline (7.01 g) in 24 ml of 2N hydrochloric acid and 115 ml of water under reflux for 3 hours, followed by neutralization with sodium bicarbonate, extraction with methylen chloride and evaporation of solvent, 11.2 g of a material are isolated which after being purified by silicagel column chromatography and crystallization from hexane gives 7.3 g (60%) of 2-(2-pyrrolidinoethoxy)ethyl 2-(7-chloro-4-quinolyl)aminobenzoate (I', R = Cl, n = 1, m = 4); m.p. 60—63°, correct elemental analysis.

IR Spectrum (KBr), $cm^{-1}$: 3290, 3000—2890, 1670, 1610, 1575, 1525, 1455, 1250, 1085, 860, 745.

Example 23

2-(2-Pyrrolidinoethoxy)ethyl 2-(7-trifluoromethyl-4-quinolyl)aminobenzoate (I', R = $CF_3$, n = 1, m = 4)

By treating 2-(2-pyrrolidinoethoxy)ethyl (7.7 g) as described in Example 22, and 4-chloro-7-trifluoromethylquinoline (8.2 g) as described in Example 22, 7.8 g (yield: 60%) of 2-(2-pyrrolidinoethoxy)ethyl 2-(7-trifluoromethyl-4-quinolyl)aminobenzoate (I', R = $CF_3$, n = 1, m = 4) are obtained; m.p. 58—61°C, correct elemental analysis.

IR Spectrum (KBr), $cm^{-1}$: 3400, 3240, 3000—2780, 1675, 1595, 1585, 1530, 1455, 1380, 1325, 1250, 1160, 1115, 750.

Example 24

2-(2-Piperidinoethoxy)ethyl 2-(7-chloro-4-quinolyl)aminobenzoate (I', R = Cl, n = 1, m = 5)

A) 2-(2-Piperidinoethoxy)ethyl anthranilate (V, n = 1, m = 4):

8.2 g of 2-(2-piperidinoethoxy)ethanol are reacted with 10.3 g of isatoic anhydride in 120 ml of toluene. 8.22 g (yield: 60%) of a yellow oil are isolated according to example 16. Then, it is purified by careful distillation in ball furnace at 190—210°C/$10^{-3}$ torr, as a viscous yellow oil; $n_D^{20}$ = 1.5528, correct C, H, N elemental analysis.

IR Spectrum (KBr), $cm^{-1}$: 3460, 3360, 3000—2700, 1685, 1615, 1290, 1240, 1155, 1100, 750.

B) Reaction of 4,7-dichloroquinoline (VI, R = Cl):

By treating 2-(2-piperidinoethoxy)ethyl anthranilate (5.85 g) as described above, and 4,7-dichloroquinoline (5.07 g), it is obtained 2-(2-piperidinoethoxy)ethyl 2-(7-chloro-4-quinolyl)aminobenzoate (I', R = Cl, n = 1, n = 5) (yield: 57%) as described in Example 22; m.p. 75—79°C, correct elemental analysis.

IR Spectrum (KBr), $cm^{-1}$: 3440, 3240, 3300—2660, 1675, 1600, 1580, 1530, 1450, 1375, 1255, 1130, 1085, 950, 745.

Example 25

2-(2-Piperidinoethoxy)ethyl 2-(7-trifluoromethyl-4-quinolyl)aminobenzoate (I', R = $CF_3$, n = 1, m = 5)

By treating 2-(2-piperidinoethoxy)ethyl anthranilate (6.12 g) as described in Example 24 and 4-chloro-7-trifluoromethylquinoline (5.92 g) as described in above examples, there is obtained 2-(2-piperidinoethoxy)ethyl 2-(7-trifluoromethyl-4-quinolyl)aminobenzoate (I', R = $CF_3$, n = 1, m = 5); m.p. 81—83°C, correct elemental analysis.

IR Spectrum (KBr), $cm^{-1}$: 3290, 3000—2700, 1675, 1610, 1580, 1530, 1455, 1250, 1150, 745.

Example 26

2-(2-Homopiperidinoethoxy)ethyl 2-(7-chloro-4-quinolyl)aminobenzoate (I', R = Cl, n = 1, m = 6)

A) 2-(2-Homopiperidinoethoxy)ethyl anthranilate (V, n = 1, m = 6):

5.64 g of isatoic anhydride are reacted with 5.02 g of 2-(2-homopiperidinoethoxy)ethanol in 100 ml of toluene, 6.96 g (yield: 85%) of a yellow oil are isolated according to Example 16; $n_D^{20}$ = 1.5505, correct C, H, N elemental analysis.

IR Spectrum (film), $cm^{-1}$: 3450, 3360, 3000—2660, 1680, 1610, 1580, 1480, 1290, 1240, 1150, 1110, 745.

B) Reaction of 4,7-dichloroquinoline (VI, R = Cl):

By treating 2-(2-homopiperidinoethoxy)ethyl anthranilate (6.12 g) as described above, and 4,7-dichloroquinoline (5.07 g) as described in Example 22, it is obtained 2-(2-homopiperidinoethoxy)ethyl 2-(7-chloro-4-quinolyl)aminobenzoate (I', R = Cl, n = 1, m = 6) (yield: 51%); m.p. 62—64°C, correct elemental analysis.

IR Spectrum (KBr), $cm^{-1}$: 3240, 3000—2700, 1675, 1600, 1570, 1520, 1445, 1245, 1080, 875, 740.

Example 27

2-(2-Homopiperidinoethoxy)ethyl 2-(7-trifluoromethyl-4-quinolyl)aminobenzoate (I', R = $CF_3$, n = 1, m = 6)

By treating 2-(2-homopiperidinoethoxy)ethyl anthranilate (6.12 g) as described in Example 26, and 4-chloro-7-trifluoromethylquinoline (5.92 g) as described in above examples, 4.51 g (yield: 45%) of 2-(2-homopiperidinoethoxy)ethyl 2-(7-trifluoromethyl-4-quinolyl)aminobenzoate (I', R = $CF_3$, n = 1, m = 6) are obtained; m.p. 53—56°C, correct elemental analysis.

IR Spectrum (KBr), $cm^{-1}$: 3260, 3000—2700, 1670, 1595, 1580, 1520, 1445, 1375, 1315, 1240, 1155, 1105, 740.

**Claims**

1. 2-Aminobenzoic acid derivatives having the general formula (I):

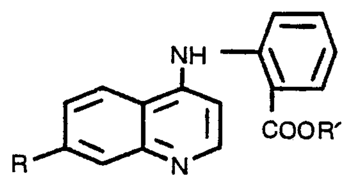

(I)

wherein

R is a halogen atom or a trifluoromethyl group,

R' represents a radical having the general formula:

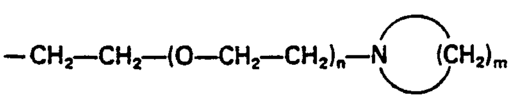

wherein

n is 0 or 1 and

m is 4, 5 or 6;

or a 1(3H)isobenzofuranon-3-yl group;

and their non-toxic addition salts.

2. A process for preparing the 2-aminobenzoic acid derivatives according to claim 1, characterized in that

A) a compound of the general formula II:

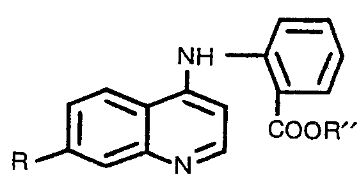

(II)

wherein R is as defined above and R'' is a $C_1$—$C_4$-alkyl group, preferably a methyl group, is reacted with a compound of the general formula III:

$$HO—R' \qquad (III)$$

wherein R' is as defined above, in an aromatic solvent at elevated temperatures in the presence of sodium or sodium hydride, preferably sodium, as catalyst, or

B) a compound of the general formula II:

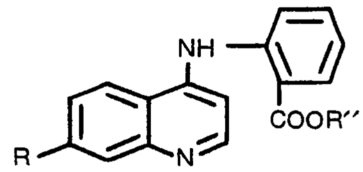

(II)

wherein R is as defined above, and R'' is a hydrogen atom, is reacted with a compound of the general formula III:

$$X—R' \qquad (III)$$

wherein X is a halogen atom, preferably a chlorine or bromine atom, and R' is as defined above, in hexamethylphosphoric triamide or N,N-dimethylformamide, preferably hexamethylphosphoric triamide, at room temperature in the presence of a tertiary amine, and

optionally the so obtained products are then converted to the corresponding addition salts.

3. A process for preparing the 2-amino benzoic acid derivatives having the general formula I':

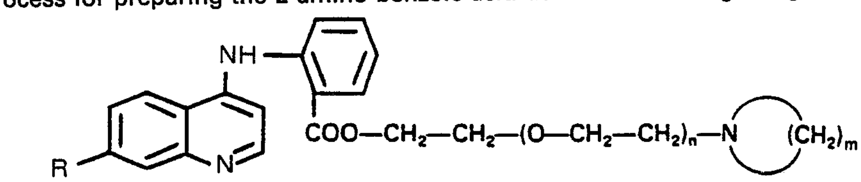

(I')

wherein R is a halogen atom or a trifluoromethyl group, n is 0 or 1 and m is 4, 5 or 6 and the addition salts thereof, characterized in that a compound of the general formula IV:

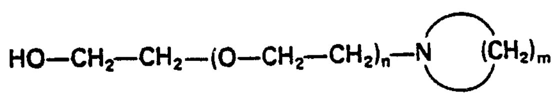

(IV)

wherein n and m are as defined above, is reacted with isatoic anhydride in an aromatic solvent at elevated temperatures to obtain a compound of the general formula V:

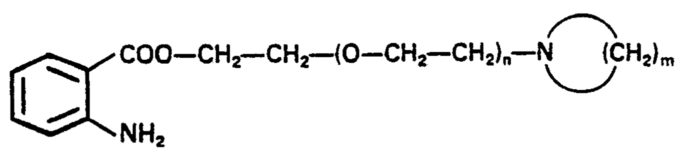

(V)

wherein n and m are as defined above, and a compound of the general formula V is then reacted with a compound of the general formula VI:

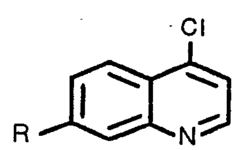

(VI)

wherein R is as defined above, in an aqueous medium previously acidified with a mineral acid, preferably HCl, at elevated temperatures, preferably under reflux, and the so obtained product is then treated with an alkali metal or alkaline earth metal carbonate or bicarbonate, and optionally the so obtained product is then converted to the corresponding addition salt.

4. A process according to claim 3, characterized in that the reaction is carried out in boiling toluene as aromatic solvent.

5. Pharmaceutical composition, comprising at least one of the compounds according to claim 1, optionally in combination with pharmaceutically acceptable carriers and adjuvants.

**Patentansprüche**

1. 2-Aminobenzoesäurederivate der allgemeinen Formel (I):

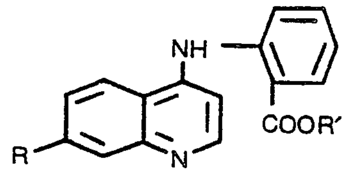

(I)

worin

R für ein Halogenatom oder eine Trifluormethylgruppe steht,

R' für einen Rest der allgemeinen Formel:

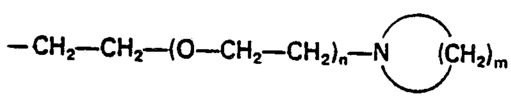

steht, worin

n für 0 oder 1 steht und

m für 4, 5 oder 6 steht;

oder für eine 1-(3H)-Isobenzofuranon-3-ylgruppe steht;

und die nicht-toxischen Additionssalze davon.

2. Verfahren zur Herstellung der 2-Aminobenzoesäurederivate nach Anspruch 1, dadurch gekennzeichnet, daß

A) eine Verbindung der allgemeinen Formel (II):

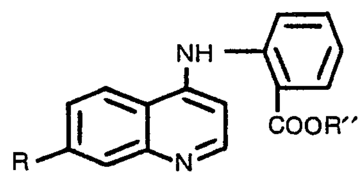

(II)

worin

R die oben erwähnten Bedeutungen besitzt und

R'' für eine $C_1$—$C_4$-Alkylgruppe, vorzugsweise eine Methylgruppe, steht,

mit einer Verbindung der allgemeinen Formel (III):

HO—R' (III)

worin R' die oben erwähnten Bedeutungen besitzt,
in einem aromatischen Lösungsmittel bei erhöhten Temperaturen in Anwesenheit von Natrium oder Natriumhydrid, vorzugsweise Natrium, als Katalysator umgesetzt wird, oder

B) eine Verbindung der allgemeinen Formel (II):

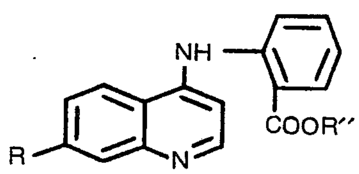

(II)

worin

R die oben erwähnten Bedeutungen besitzt, und

R'' für ein Wasserstoffatom steht,

mit einer Verbindung der allgemeinen Formel (III):

X—R' (III)

worin

X für ein Halogenatom, vorzugsweise ein Chlor- oder Bromatom, steht, und

R' die oben angegebenen Bedeutungen besitzt,

in Hexamethylphosphor-triamid oder N,N-Dimethylformamid, vorzugsweise Hexamethylphosphortriamid, bei Raumtemperatur in Anwesenheit eines tertiären Amins umgesetzt wird,
und gegebenenfalls die auf diese Weise erhaltenen Produkte anschließend in die entsprechende Additionssalze überführt werden.

3. Verfahren zur Herstellung der 2-Aminobenzoesäurederivate der allgemeinen Formel (I'):

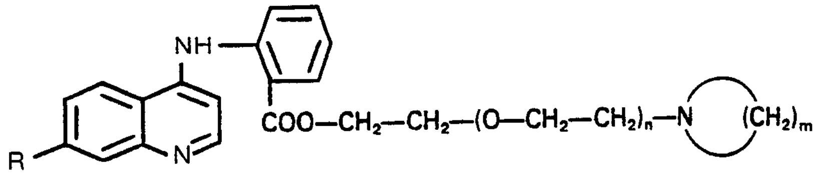

(I')

worin
R für ein Halogenatom oder eine Trifluormethylgruppe steht,
n für 0 oder 1 und
m für 4, 5 oder 6 steht,
und der Additionssalze davon, dadurch gekennzeichnet, daß eine Verbindung der allgemeinen Formel (IV):

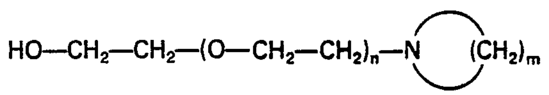

(IV)

worin n und m die oben gegebenen Bedeutungen besitzen, mit Isatosäureanhydrid in einem aromatischen Lösungsmittel bei erhöhten Temperaturen umgesetzt wird, wobei man eine Verbindung der allgemeinen Formel (V):

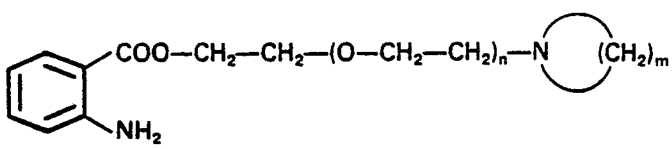

(V)

erhält, worin n und m die oben angegebenen Bedeutungen besitzen,
und eine Verbindung der allgemeinen Formel (V) dann mit einer Verbindung der allgemeinen Formel (VI):

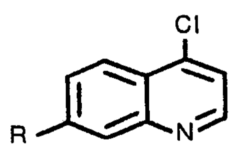

(VI)

worin R die oben angegebenen Bedeutungen besitzt, in einem vorher mit einer Mineralsäure, vorzugsweise HCl, angesäureten wäßrigen Medium bei erhöhten Temperaturen, vorzugsweise unter Rückfluß, umgesetzt wird, und das auf diese Weise erhaltene Produkt anschließend mit einem Alkalimetall- oder Erdalkalimetallcarbonat- oder -bicarbonat behandelt wird, und gegebenenfalls das auf diese Weise erhaltene Produkt dann in das entsprechende Additionssalz überführt wird.

4. Verfahren nach Anspruch 3, dadurch gekennzeichnet, daß die Reaktion in siedendem Toluol als aromatisches Lösungsmittel ausgeführt wird.

5. Pharmazeutisches Mittel, das mindestens eine der Verbindungen nach Anspruch 1 gegebenenfalls in Kombination mit pharmazeutisch akzeptablen Trägern und Adjuvanzien enthält.

**Revendications**

1. Dérivés de l'acide 2-aminobenzoïque répondant à la formule générale (I):

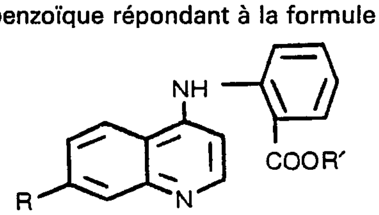

(I)

dans laquelle
R est un atome d'halogène ou un groupe trifluorométhyle,
R' représente un radical répondant à la formule générale:

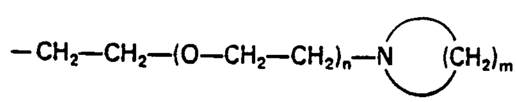

dans laquelle
n est 0 ou 1 et
m est 4, 5, ou 6;
ou un groupe 1(3H)-isobenzofuranon-3-yle;
et leurs sels d'addition non toxiques.

2. Procédé pour préparer les dérivés de l'acide 2-aminobenzoïque selon la revendication 1, caractérisé en ce que

A) on fait réagir un composé répondant à la formule générale II:

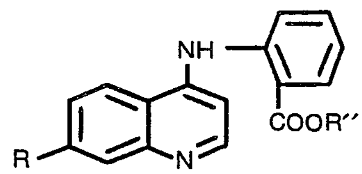

(II)

dans laquelle R est tel que défini ci-dessus et R'' est un groupe alkyle en $C_1$ à $C_4$, de préférence un groupe méthyle, avec un composé répondant à la formule générale III:

$$HO—R' \qquad (III)$$

dans laquelle R' est tel que défini ci-dessus, dans un solvant aromatique à températures élevées en présence de sodium ou d'hydrure de sodium, de préférence de sodium, comme catalyseur, ou

B) on fait réagir un composé répondant à la formule générale II:

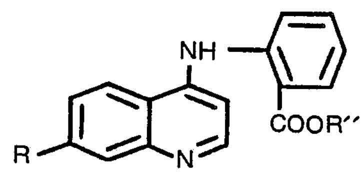

(II)

dans laquelle R est tel que défini ci-dessus, et R'' est un atome d'hydrogène avec un composé répondant à la formule générale III:

$$X—R' \qquad (III)$$

dans laquelle X est un atome d'halogène, de préférence un atome de chlore ou de brome, et R' est tel que défini ci-dessus, dans de l'hexaméthylphosphotriamide ou du N,N-diméthylformamide, de préférence de l'hexaméthylphosphotriamide, à la température ambiante, en présence d'une amine tertiaire et,
on transforme alors si on le désire les produits ainsi obtenus en les sels d'addition correspondants.

3. Procédé pour préparer les dérivés de l'acide 2-aminobenzoïque répondant à la formule générale I':

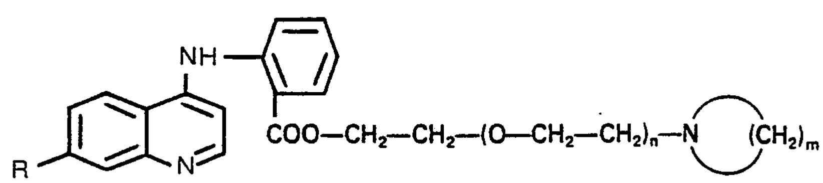

(I')

dans laquelle R est un atome d'halogène ou un groupe trifluorométhyle, n est 0 ou 1 et m est 4, 5 ou 6, et leurs sels d'addition, caractérisé en ce que: on fait réagir un composé répondant à la formule générale IV:

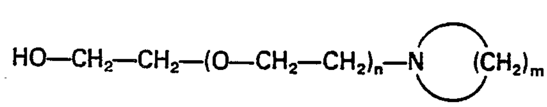

(IV)

dans laquelle n et m sont tels que définis ci-dessus, avec de l'anhydride isatoïque dans un solvant aromatique, à températures élevées pour obtenir un composé répondant à la formule générale V:

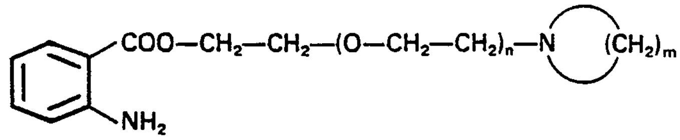

(V)

dans laquelle n et m sont tels que définis ci-dessus, et on fait alors réagir un composé répondant à la formule générale V avec un composé répondant à la formule générale VI:

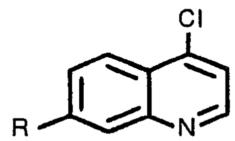

(VI)

dans laquelle R est tel que défini ci-dessus, dans un milieu aqueux acidifié au préalable avec un acide minéral, de préférence HCl, à températures élevées, de préférence sous reflux, puis on traite le produit ainsi obtenu par un carbonate ou bicarbonate de métal alcalin ou de métal alcalino-terreux et on transforme alors, si on le désire, le produit ainsi obtenu en le sel d'addition correspondant.

4. Procédé selon la revendication 3, caractérisé en ce que la réaction est effectuée dans du toluène bouillant comme solvant aromatique.

5. Composition pharmaceutique, comprenant au moins un des composés selon la revendication 1, associés si on le désire à des supports et adjuvants pharmaceutiquement acceptables.